# EUROPEAN PATENT APPLICATION

(11) **EP 1 281 967 A2**
(43) Date of publication of application: **05.02.2003**
(21) Application number: 02017951.1
(22) Date of filing: 30.11.1999
(51) Int. Cl.: G01N 33/543, C12Q 1/68, B01L 3/02

(54) **Biochip and method for producing the same**

(30) Priority: 01.12.1998 JP 34160498
(62) Divisional of application: 99123786.8
(71) Applicant: Hitachi Software Engineering Co., Ltd., Yokohama-shi, Kanagawa 231-8475 (JP)
(72) Inventor: Ito, Toshiaki, c/o Hitachi Software Eng. Co.,Ltd., Yokohama-shi, Kanagawa 231-8475 (JP); Yamamoto, Kenji, c/o Hitachi Software Eng. Co.,Ltd, Yokohama-shi, Kanagawa 231-8475 (JP); Watanabe, Toshimasa, Hitachi Software Eng. Co.,Ltd, Yokohama-shi, Kanagawa 231-8475 (JP); Yurino, Noriko, c/o Hitachi Software Eng. Co.,Ltd., Yokohama-shi, Kanagawa 231-8475 (JP)
(74) Representative: Liesegang, Roland, Dr.-Ing.

(57) **Abstract**

A biochip comprising probes spotted on a plate at a plurality of positions by using a binding agent for binding the probes to the plate, wherein the binding agent is locally spotted at positions where the probes are spotted.

## Description

### FIELD OF THE INVENTION

The present invention relates to a biochip comprising a plate spotted with various probes.

### BACKGROUND OF THE INVENTION

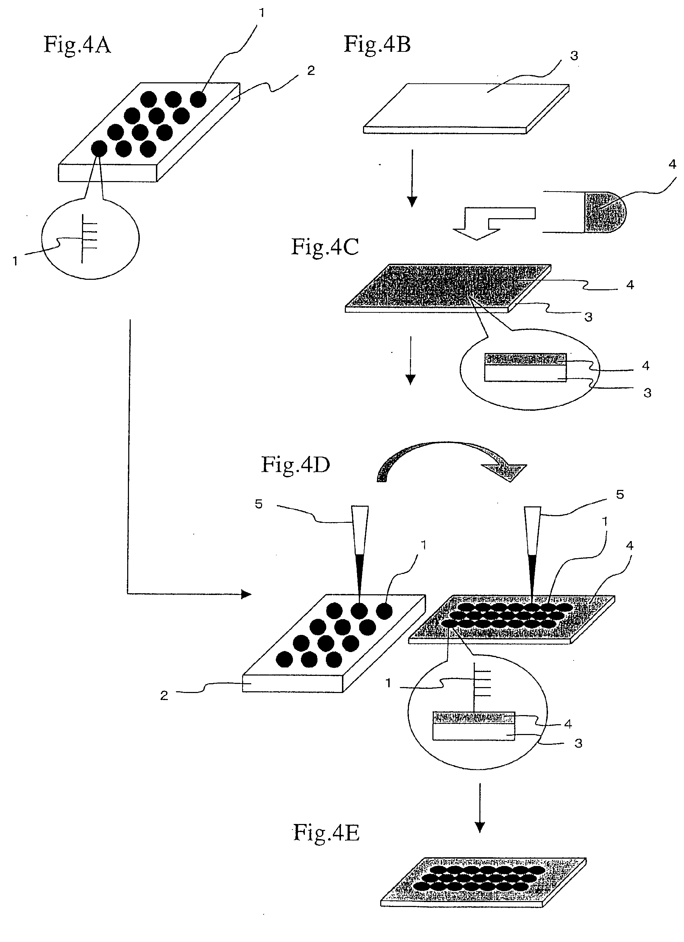

Conventionally, biochips are produced by spotting various biopolymer probes such as DNAs, RNAs and proteins on a plate (e.g., a glass plate). Figures 4A to 4E are diagrams for illustrating the principle of such conventional technique. First, a microplate 2 containing various probe DNAs 1 (Figure 4A) and a glass plate 3 (Figure 4B) are prepared. As shown in Figure 4C, the surface of the glass plate 3 is coated with poly-1-lysine binding agent 4 for binding the DNAs 1 to the glass plate 3. Thereafter, each of the probe DNAs 1 in the microplate 2 is transferred by a pin 5 and spotted onto the glass plate 3 coated with the poly-1-lysine binding agent 4 (Figure 4D). This process is repeated for all of the probe DNAs 1 in the microplate 2, thereby producing a biochip shown in Figure 4E. In such manner, the binding agent for binding DNA to the glass plate is conventionally coated on the entire surface of the plate before spotting the DNAs on the plate.

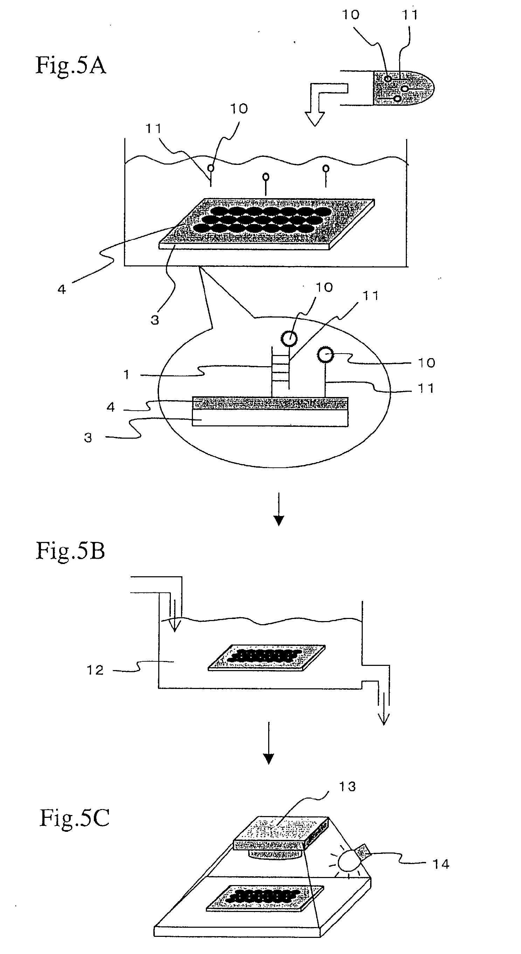

Figures 5A to 5C are diagrams for illustrating the principle of hybridization using the biochip. Referring to Figure 5A, sample DNA 11 labeled with a fluorescent substance 10 is hybridized in a hybridization solution with the probe DNAs 1 that are spotted onto the glass plate 3 of the biochip via the binding agent 4. The hybridization solution contains formaldehyde, SSC (NaCl, trisodium citrate), SDS (sodium dodecyl sulfate), EDTA (ethylenediamidetetraacetic acid), distilled water and the like where the mixing ratio depends on the characteristics of the DNA used.

When the sample DNA 11 is complementary to any one of the probe DNAs 1 on the biochip, it binds to that DNA on the biochip and forms a duplex. The sample DNA 11 does not bind to probe DNAs that are not complementary thereto. However, the sample DNA 11 may bind to the binding agent 4 coating the glass plate 3, thereby remaining as garbage.

As shown in Figure 5B, the glass plate 3 of the biochip hybridized with the sample DNA 11 is washed in water 12 to remove the sample DNA 11 that is not bound to the probe DNAs 1. Referring to Figure 5C, the fluorescent substance 10 labeling the sample DNA 11 bound to the probe DNA 1 is excited with light from a lamp 14. The fluorescent light emanated from the fluorescent substance 10 is detected by an optical sensor 13 such as a CCD to detect the presence of hybridization.

In a laboratory, the sample DNA 11 is poured onto the biochip to allow hybridization with the probe DNAs 1 spotted on the biochip followed by detection of the probe DNA bound by the sample DNA 11. Following the hybridization and prior to the detection, the biochip is washed with water to remove the sample DNA 11 that did not bind to the probe DNAs. However, since the entire surface of the glass plate 3 is coated with the binding agent 4 for binding the probe DNA to the glass plate 3, sample DNA 11 adheres to the binding agent area of the glass plate 3 where the probe DNAs 1 are not located. The sample DNA 11 bound to the binding agent 4 cannot be removed from the glass plate 3 by washing with water. Such remainder sample DNA 1 is detected as noise upon detection, rendering the detection sensitivity poor. In other words, some of the sample DNA 11 that is not specific to the probe DNA binds to and remains on the biochip via the binding agent 4 as garbage. When the fluorescent substance 10 labeling the sample DNA 11 bound to the binding agent 4 is excited, the fluorescent light therefrom is detected as noise, whereby S/N (signal-to-noise) ratio is lowered.

The present invention aims to solve this problem, and provides a biochip in which sample DNA does not bind to area of the plate where the probes are not located. The present invention also provides a method for producing such biochip.

### SUMMARY OF THE INVENTION

In order to accomplish the above object, the present invention provides a binding agent for binding probes on a plate only where the probes are to be spotted. Since no binding agent is provided on the portions of the plate where the probes are not to be spotted, the sample DNA that does not bind to the probe upon hybridization can be removed away from the biochip by washing with water. Therefore, noise produced upon detection can be eliminated and thus the S/N ratio can be enhanced for high sensitivity.

A biochip according to the present invention includes probes spotted on a plate at a plurality of positions by using a binding agent for binding the probes to the plate, wherein the binding agent is locally spotted at positions where the probes are spotted.

In a preferred embodiment of the inventive biochip the material of the plate is selected from the group comprising glass, nylon membranes, silicone wafer, polyimide resin and polymer plastic.

In a further preferred embodiment of the inventive biochip the binding agent is selected from the group comprising poly-1-lysine, carbodiimide and silylation-coating.

A method for producing a biochip by spotting probes on a plate by using a binding agent for binding the probes to the plate according to the present invention includes a step of spotting mixtures of respective probes and the binding agent on the plate.

An alternative method for producing a biochip by spotting probes on a plate according to the present invention includes the steps of: spotting a binding agent for binding the probes to the plate at positions where the probes are to be spotted; and spotting the probes on the plate at positions where the binding agent is spotted.

The plate used in the inventive methods may be made of a material selected from the group comprising glass, nylon membranes, silicone wafer, polyimide resin and polymer plastic.

In a preferred embodiment of the inventive methods the binding agent is selected from the group comprising poly-1-lysine, carbodiimide and silylation-coating.

The probes are preferably spotted by using the inventive pin.

The problem underlying the present invention is also solved by a pin used for spotting a probe on a plate, wherein a tip of the pin comprises at least a recess.

In one embodiment of the inventive pin the recess is of a concave shape.

In a second embodiment of the inventive pin the recess comprises at least one groove.

In a third embodiment the recess comprises a radially-shaped groove.

According to the invention, a pin used for spotting a probe on a plate has a tip provided with at least one groove. For example, the groove may be a radially-shaped groove such as a cross-shaped groove.

This specification includes all or part of the contents as disclosed in the specification and/or drawings of Japanese Patent Application No. 10-341604, which is a priority document of the present application.

### BRIEF DESCRIPTION OF THE DRAWINGS

Figures 1A to 1E are schematic diagrams showing the principle of one embodiment of the present invention;
Figures 2A to 2E are diagrams showing the principle of another embodiment of the present invention;
Figures 3A to 3C are diagrams for illustrating the principle of the hybridization and detection using the biochip of the invention;
Figures 4A to 4E are diagrams for illustrating the principle of a method for producing a conventional biochip;
Figures 5A to 5C are diagrams for illustrating the principle of hybridization and detection using the conventional biochip; and
Figures 6A to 6C are schematic diagrams showing shapes of a tip (i.e., a portion where probes are to be contacted and carried) of a pin according to the invention.

### DETAILED DESCRIPTION OF THE INVENTION

Hereinafter, the present invention will be described in more detail by way of examples with reference to the accompanying drawings. In the examples, DNA is used as a probe although the probe is not limited thereto, and RNA or protein may also be used as a probe. Although a glass plate is used in the examples, a nylon membrane or the like may also be used.

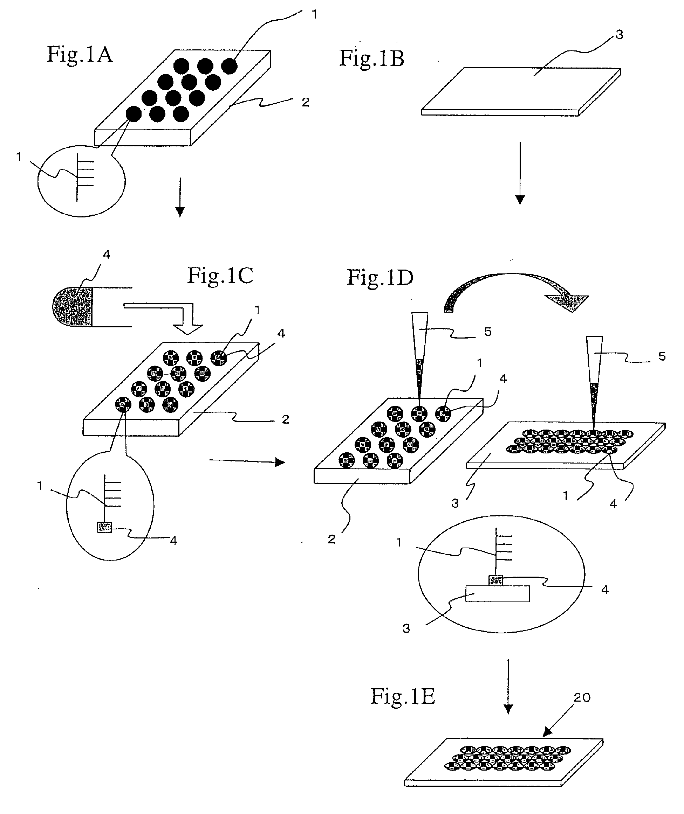

Figures 1A to 1E are schematic diagrams showing the principle of a first embodiment of the present invention. As shown in Figure 1A, a microplate 2 contains various probe DNAs 1. A plate 3 to be incorporated into the biochip shown in Figure 1B is made of glass. Referring to Figure 1C, a binding agent 4 for binding DNA to glass is dispensed into each well of the microplate 2 to be mixed therein with each of the probe DNAs 1. The binding agent 4 may be, for example, poly-1-lysine or carbodiimide.

Then, as shown in Figure 1D, each of the mixtures of the binding agent 4 and the probe DNAs 1 is suctioned by a pin 5 (or contacted and carried by the tip of the pin 5) and spotted onto the plate 3. This process is repeated for all of the probe DNAs 1 in the microplate 2, thereby producing a biochip 20 shown in Figure 1E in which the binding agent 4 is present only at the desired portions and is not present at portions where there is no probe.

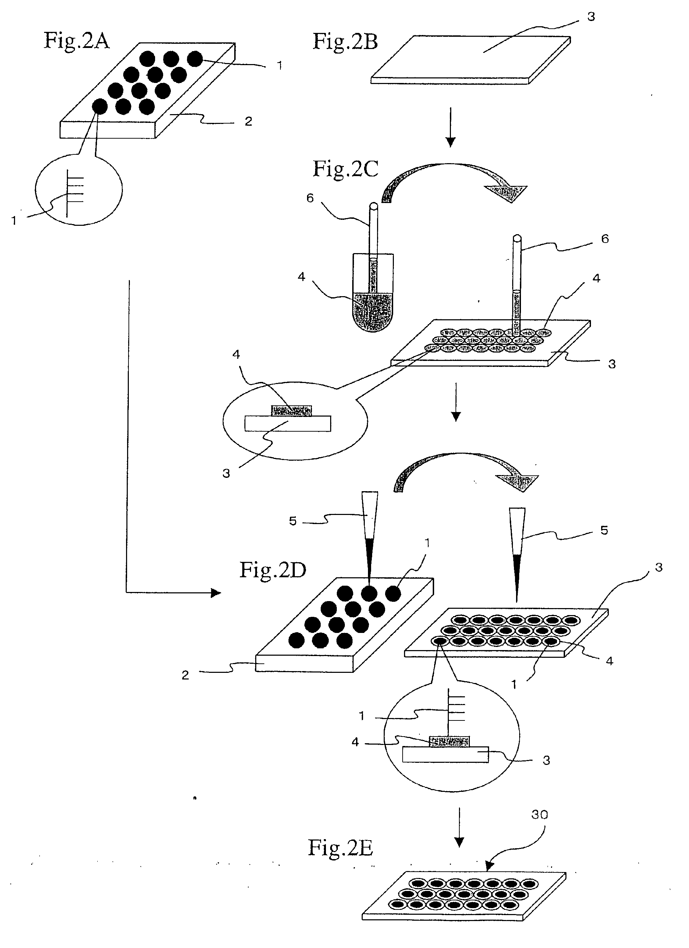

Figures 2A to 2E are diagrams showing the principle of a second embodiment of the present invention. A microplate 2 containing various probe DNAs 1 (Figure 2A) and a plate 3 made of glass (Figure 2B) are prepared. As shown in Figure 2C, a binding agent is suctioned by, for example, a capillary tube 6 and applied on the glass plate 3 at positions where the probe DNAs are to be spotted. Then, as shown in Figure 2D, the probe DNAs 1 in the microplate 2 are suctioned with the pin 5 (or is contacted and carried by the tip of the pin 5) and spotted onto the plate 3. This process is repeated for all of the probe DNAs 1 in the microplate 2, thereby producing a biochip 30 in which the binding agent 4 is not provided on portions other than portions where the probe DNAs 1 are present (Figure 2E).

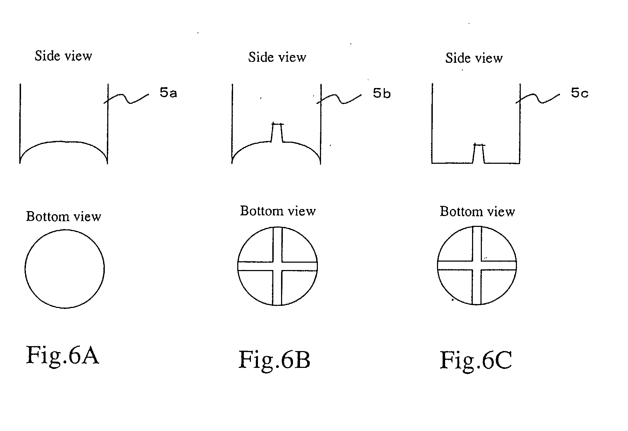

Figures 6A to 6C are schematic diagrams showing shapes of a tip (i.e., a portion where probes are to be contacted) of a pin 5 according to the invention. Figure 6A shows a pin 5a with a concave tip. A pin 5b shown in Figure 6B has a concave tip with a cross-shaped groove. The concave shape of the tip of the pin allows the probe solution to be carried by surface tension by simply dipping the pin in the solution. The depth of the concave is optional. The amount of the DNA carried with the pin 5a or 5b with the concave tip is about 10 times or more the amount carried with a conventional pin with a flat tip. A pin 5c shown in Figure 5C has a flat tip with a cross-shaped groove. The amount of the DNA carried with this pin 5c is also higher than that carried with the conventional flat tip.

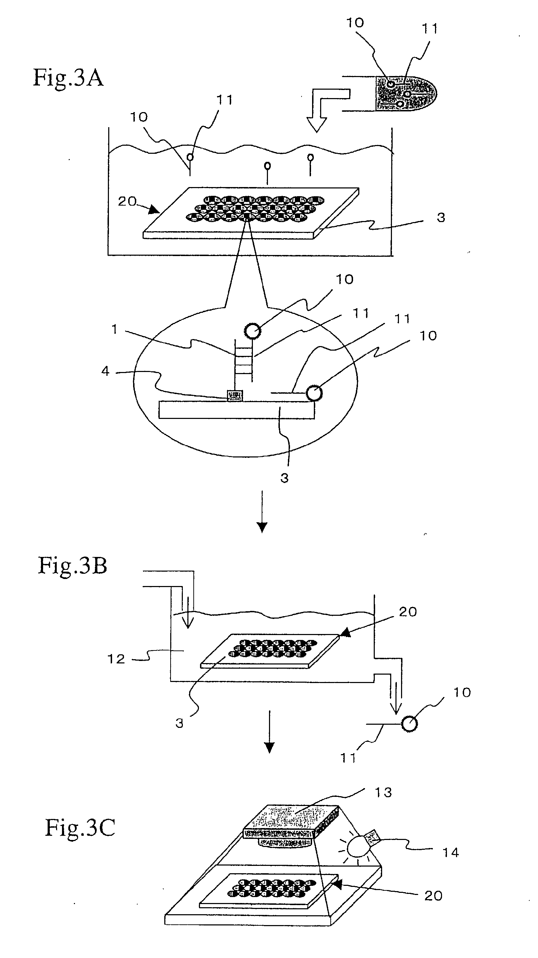

Figures 3A to 3C are diagrams for illustrating the principle of hybridization using the biochip 20 of the invention. Referring to Figure 3A, a sample DNA 11 labeled with a fluorescent substance 10 is placed together with the biochip 20 in a hybridization solution for hybridization. The probe DNAs 1 are spotted on the glass plate 3 via the binding agent 4 in the biochip 20. The hybridization solution contains formaldehyde, SSC (NaCl, trisodium citrate), SDS (sodium dodecyl sulfate), EDTA (ethylenediamidetetraacetic acid) and distilled water where the mixing ratio differs depending on the characteristic of the DNA used.

When the sample DNA 11 and any one of the probe DNAs 1 on the biochip 20 are complementary to each other, both DNAs bind to each other and form a duplex. On the other hand, when the sample DNA 11 and any one of the probe DNAs 1 are not complementary to each other, the sample DNA 11 does not bind to that probe DNA 1 and remain as garbage. As shown in Figure 3B, the sample DNA 11 labeled with the fluorescent substance 10 remaining on the glass plate 3 is washed away in water 12. Since the binding between the glass and the DNA is weak, the remaining garbage sample 11 that is not bound to the probe DNAs 1 is removed away. Referring to Figure 3C, the fluorescent substance 10 labeling the sample DNA 11 bound to the probe DNA 1 is excited with light from a lamp 14. The fluorescent light emanated from the fluorescent substance 10 is detected by an optical sensor 13 such as a CCD to detect the presence of hybridization. Since there is no garbage sample DNA left on the biochip 20, the S/N ratio upon detection is enhanced.

According to the present invention, a biochip can be produced in which a binding agent is locally spotted only where probes are to be spotted. Thus, the detection sensitivity upon reading the biochip can be enhanced.

All publications, including patent and patent application cited herein are incorporated herein by reference in their entirety.

The features disclosed in the foregoing description, in the claims and/or in the accompanying drawings may, both separately and in any combination thereof, be material for realising the invention in diverse forms thereof.

## Claims

1. A biochip comprising probes spotted on a plate at a plurality of positions by using a binding agent for binding the probes to the plate, wherein the binding agent is locally spotted at positions where the probes are spotted.

2. The biochip according to claim 1 wherein the material of the plate is selected from the group comprising glass, nylon membranes, silicone wafer, polyimide resin and polymer plastic.

3. The biochip according to claim 1 or 2, wherein the binding agent is selected from the group comprising poly-1-lysine, carbodiimide and silylation-coating.

4. A method for producing a biochip by spotting probes on a plate by using a binding agent for binding the probes to the plate, the method comprising a step of spotting mixtures of respective probes and the binding agent on the plate.

5. A method for producing a biochip by spotting by spotting probes on a plate, the method comprising the steps of:
spotting a binding agent for binding the probes to the plate at positions where the probes are to be spotted; and
spotting the probes on the plate at positions where the binding agent is spotted.

6. The method for producing a biochip according to claim 4 or 5, wherein the plate is made of a material which is selected from the group comprising nylon membranes, glass, silicone wafer, polyimide resin and polymer plastic.

7. The method according to any of claims 4 to 6, wherein the binding agent is selected from the group comprising poly-1-lysine, carbodiimide and silylation-coating.
